# EUROPEAN PATENT APPLICATION

(11) **EP 4 134 454 A1**
(43) Date of publication of application: **15.02.2023**
(21) Application number: 21893921.3
(22) Date of filing: 17.11.2021
(51) Int. Cl.: C12Q 1/6886

(54) **REAGENT COMBINATION AND KIT FOR DETECTING LIVER CANCERS, AND USE THEREOF**

(30) Priority: 17.11.2020 CN 202011288235; 17.11.2020 CN 202011290799; 17.11.2020 CN 202011290484
(71) Applicant: Sansure Biotech Inc., Changsha, Hunan 410205 (CN)
(72) Inventor: DAI, Lizhong, Changsha, Hunan 410205 (CN); CHEN, Ming, Changsha, Hunan 410205 (CN); GUO, Xinwu, Changsha, Hunan 410205 (CN); HONG, Mei, Changsha, Hunan 410205 (CN); LIU, Jia, Changsha, Hunan 410205 (CN); LIU, Rangjiao, Changsha, Hunan 410205 (CN)
(74) Representative: Grünecker Patent- und Rechtsanwälte PartG mbB
(86) International application number: PCT/CN2021/131103
(87) International publication number: WO 2022/105774

(57) **Abstract**

Provided is a reagent combination for detecting a liver cancer. The reagent combination includes any one of the following detection reagents: 1) a detection reagent for detecting the methylation level of at least one of the following methylation sites of the PAK1 gene: cg17202086, cg26996201, and cg18309286; 2) a detection reagent for detecting the methylation level of at least one of the following methylation sites of the OTX1 gene: cg23229261 and cg10122865; and 3) a detection reagent for detecting the methylation level of methylation site cg16657538 of the ZNF397OS gene. Further provided are use of the reagent combination, and a kit including the reagent combination. By using the reagent combination of the present invention, a liver cancer may be detected clinically in a tissue sample with fewer markers, and may be detected sensitively and specifically by detecting methylation sites, and the cost and time are saved; thereby in clinic a liver cancer may be sensitively and specifically detected in the early stage of liver malignancy.

## Description

### CROSS-REFERENCE TO RELATED APPLICATIONS

This application is based on the following Chinese patent applications: CN202011288235.7 with the filing date of November 17, 2020; CN202011290484.X with the filing date of November 17, 2020; and CN202011290799.4 with the filing date of November 17, 2020; and claims for the priorities of these Chinese patent applications whose entire content are incorporated herein by reference.

### TECHNICAL FIELD

The invention belongs to the field of molecular biology detection, in particular to the field of liver cancer detection, and more particularly to the detection of the methylation level of liver cancer gene markers.

### BACKGROUND

Liver cancer is one of the most common and deadly diseases in the world, and is a very harmful malignant tumor. Up to 50% of the world's liver cancer patients are in China, liver cancer is the fourth most common malignant tumor and the second leading cause of tumor death in China. According to the data of "Analysis on China Malignant Cancer Epidemic in 2015" released in 2019, the incidence of liver cancer in China was about 370,000 in 2015; the death of liver cancer was about 326,000, this disease is a serious threat to the life and health of the Chinese people. The onset of liver cancer is insidious, and there are no specific symptoms in the early stage. Most liver cancer patients are in the middle or late stages at the time of treatment, thus the surgical cure rate is low and the survival period is short.

At present, serum alpha fetal protein (AFP) and ultrasonography are commonly used and important methods for clinical diagnosis of liver cancer, but their sensitivity and specificity are not ideal. The sensitivity of AFP screening may reach only 40-60%, and the AFP level of many patients with early-stage liver cancer is always maintained at a normal level; a considerable number of patients with liver disease also have abnormally elevated AFP; ultrasound detection is very dependent on instruments and manual operations, it is affected by the distribution of medical resources and the experience of doctors; contrast-enhanced ultrasound and puncture have disadvantages such as complicated operations and trauma, and are not suitable for early screening and early diagnosis. Finding accurate, stable and effective molecular markers of liver cancer is of great significance for early diagnosis and early treatment of liver cancer.

The detection of tumor markers is a method developed in recent years for detecting diseases, and finding accurate, stable and effective molecular markers of liver cancer is of great significance for the early diagnosis and early treatment of liver cancer. With the deepening of the scientific community's understanding of tumors, more and more studies have confirmed that changes in the epigenetic level of cells are the key events in the occurrence and development of tumors. DNA methylation, histone modification and abnormal expression of miRNA are all epigenetic changes, and the core stage of tumorigenesis is also related to abnormal methylation of DNA. The detection of DNA methylation has good stability, and is easy to detect; the abnormality of DNA methylation is often associated with the progress of cancer, and is the most potential marker for early tumor screening. At present, there are auxiliary diagnostic products for detecting cancers (such as colorectal cancer, and gastric cancer) based on detection of DNA methylation have been certified by the FDA or CFDA, but no screening products for detecting liver cancer is approved for marketing.

Many studies look for markers for early diagnosis of liver cancer by detecting tumor cells or DNA methylation changes in peripheral blood. A clinical study of early-stage liver cancer screening based on methylation detection by Exact Sciences in the United States shows that, in a clinical trial of 135 HCC (hepatocellular carcinoma) cases and 308 controls, this blood test reaches a sensitivity of 71% and specificity of 90% in the diagnosis of early-stage HCC patients. The detection of this project has been recognized by FDA as a breakthrough medical device, but its clinical research is mainly based on European and American populations and has the defect of low sensitivity; in Chinese patent CN107164508A, liver cancer is detected by detecting the content of 5-hydroxymethylcytosine in 9 genes, this detection has relatively good performance with a sensitivity of 90% and specificity of 91.3%. However, the samples in this group do not include people with high risk of liver cancer such as liver cirrhosis and hepatitis, so there may be a shortage of low specificity.

Therefore, in this field there is a need for a diagnostic product for liver cancer with high sensitivity and high specificity based on DNA methylation detection, which may detect liver cancer at an early-stage.

### SUMMARY

The present invention collects and constructs big data sets of liver cancer-related methylation in TCGA and GEO databases, and uses bioinformatics methods to screen out DNA methylation markers of liver cancer with potential for application and development. Through a large number of studies, the applicant found that the methylation level of the methylation sites of ZNF397OS gene is closely related to liver cancer. Before that, ZNF397OS is generally considered to be a zinc finger protein in the art. Studies have shown that ZNF397OS has the function of regulating the activity of DNA-binding transcription factors, and there is no literature reporting that it is associated with liver cancer. In addition, the applicant also found that the methylation levels of the methylation sites of the OTX1 gene and the PAK1 gene are closely related to liver cancer.

In a first aspect, the present invention provides a reagent combination for detecting a liver cancer, including any one of the following detection reagents:
1) a detection reagent for detecting the methylation level of at least one of the following methylation sites of the PAK1 gene: cg17202086, cg26996201, and cg18309286;
2) a detection reagent for detecting the methylation level of at least one of the following methylation sites of the OTX1 gene: cg23229261 and cg10122865; and
3) a detection reagent for detecting the methylation level of cg16657538 methylation site of ZNF397OS gene.

In some particular embodiments, when the above reagent combination includes the above detection reagent 1), further, the reagent combination includes a detection reagent for detecting the methylation level of at least two of the following methylation sites of the PAK1 gene: cg17202086, cg26996201, and cg18309286. more further, the reagent combination includes a detection reagent for detecting the methylation level of the following three methylation sites of the PAK1 gene: cg17202086, cg26996201, and cg18309286.

In some particular embodiments, when the above reagent combination includes the above detection reagent 1), the reagent combination is used for detecting the methylation level in the following region:

CCGTGTCACCACTCTCCGAAAGCCGGCGGAGATCCCCCAGAACCCGGCCCAGCTA
GGGGAGCCCCAGGCAGGGCCGGCATTACCTGGTGGGGAAGGCTAGCCCCGGCATCTCT
GGCGATTGTTTCCGTCGCAGAGCCGCACCTCTCCCTGCCCAACCGGGTACCCACAAGGC
CCCTGCTCCACCGGCTCCGCTCGATTCAGCTGGGCTGGGCTCCTGGCAGCTCAGCCGCC
AGCTCCCGAGGTGCAGCACGCTCGCGCTCTCGCAGGGCGGCAGCGAAGGCGCCGCGTC
GGGGTTGGCGAAACAGAAGGGGCGGGGGAAGAAAGGAGGGAAGGAGGCGGGAGGAA
GCCGCGCG (SEQ ID NO: 1).

By using the reagent combination of the present invention, in a tissue sample, a liver cancer may be predicted with a specificity of at least 0.360, a sensitivity of 0.843, and an area under the curve of 0.8194; and in a cell-free DNA sample of plasma, a liver cancer may be predicted with a specificity of at least 0.8511, a sensitivity of 0.7460, and an area under the curve of 0.828. The reagent combination of the present invention may detect a liver cancer clinically with high sensitivity and good specificity by fewer markers, and the cost and time are saved; a liver cancer may be sensitively and specifically detected in the early stage of liver malignancy.

In some particular embodiments, when the above reagent combination includes the above detection reagent 1), the reagent combination further includes a detection reagent for detecting the methylation level of at least any one of the following genes:
GRASP, ZNF397OS, PPFIA1, and OTX1.

When the above reagent combination includes the above detection reagent 1), the reagent combination further includes a detection reagent for detecting the methylation level of at least any two of the following genes:
GRASP, ZNF397OS, PPFIA1, and OTX1.

When the above reagent combination includes the above detection reagent 1), the reagent combination further includes a detection reagent for detecting the methylation level of at least any three of the following genes:
GRASP, ZNF397OS, PPFIA1, and OTX1.

When the above reagent combination includes the above detection reagent 1), the reagent combination further includes a detection reagent for detecting the methylation level of the following four genes:
GRASP, PAK1, PPFIA1, and OTX1.

In some particular embodiments, when the above reagent combination includes the above detection reagent 2), further, the reagent combination includes a detection reagent for detecting the methylation level of the following two methylation sites of the OTX1 gene: cg23229261 and cg10122865. more further, the reagent combination also includes a detection reagent for detecting the methylation level of the methylation site cg21472506 of the OTX1 gene.

In some particular embodiments, when the above reagent combination includes the above detection reagent 2), in a particular embodiment, the reagent combination is used for detecting the methylation level in the following region:

CGATTTTGCTCCACGCCTGCCGGCCAGAGCCTCCCGGCGTTTCTTCCGCCCCAGCG
GAGTGCGCTGGGGCGCGCCAGGGCTAGGCCCGCCGGAGGAGCGCGTCCCCAGCCTTCC
GCGCACAGAGCCGCATCCCGCCCCGCCCTGCGCTGGACTGGTTCAAGCTTCCGCCTCG
(SEQ ID NO: 2).

By using the reagent combination of the present invention, in a tissue sample, a liver cancer may be predicted with a specificity of at least 0.846, a sensitivity of 0.860, and an area under the curve of 0.914; and in a cell-free DNA sample of plasma, a liver cancer may be predicted with a specificity of at least 0.8298, a sensitivity of 0.778, and an area under the curve of 0.8823. The reagent combination of the present invention may detect a liver cancer clinically with high sensitivity and good specificity with fewer markers, and the cost and time are saved; a liver cancer may be sensitively and specifically detected in the early stage of liver malignancy.

In some particular embodiments, when the above reagent combination includes the above detection reagent 2), the reagent combination further includes a detection reagent for detecting the methylation level of at least any one of the following genes:
GRASP, PAK1, PPFIA1, and ZNF397OS.

When the above reagent combination includes the above detection reagent 2), the reagent combination further includes a detection reagent for detecting the methylation level of at least any two of the following genes:
GRASP, PAK1, PPFIA1, and ZNF397OS.

When the above reagent combination includes the above detection reagent 2), the reagent combination further includes a detection reagent for detecting the methylation level of at least any three of the following genes:
GRASP, PAK1, PPFIA1, and ZNF397OS.

When the above reagent combination includes the above detection reagent 2), the reagent combination further includes a detection reagent for detecting the methylation level of the following four genes:
GRASP, PAK1, PPFIA1, and ZNF397OS.

In some particular embodiments, when the above reagent combination includes the above detection reagent 3), the reagent combination is used for detecting the methylation level of the following region:

CGCCCCACTCACCCTTCGCTCTACCGGCGGCGGCGGGAACCCACCCCCGGGAGCG
AGAACAATGCCCGGCCGCACGCGCGCCGGAAGTGGGAGAGTGCCCCTCTAGGAGCCCG
GAGGACCGCAGCTCTGTGGCAGGCGCGGGTCGTGTCTCGCAGGAGGGGCGCGGGTCCG
GCTCAGACCTGGCGGGGGCATCGCAGAGTACAAGCGGTTGACGCG (SEQ ID NO: 3).

By using the reagent combination of the present invention, a liver cancer may be detected clinically in a tissue sample with a sensitivity of 0.910, a specificity of 0.950, and an area under the curve of 0.941; and in a cell-free DNA sample with a sensitivity of 0.7619, a specificity of 0.9574, and an area under the curve of 0.8948 by fewer markers. A liver cancer may be sensitively and specifically detected by detecting one methylation site of the gene, and the cost and time are saved; thereby in clinic a liver cancer may be sensitively and specifically detected in the early stage of liver malignancy.

In some particular embodiments, when the above reagent combination includes the above detection reagent 3), the reagent combination further includes a detection reagent for detecting the methylation level of at least any one of the following genes:
GRASP, PAK1, PPFIA1, and OTX1.

When the above reagent combination includes the above detection reagent 3), the reagent combination further includes a detection reagent for detecting the methylation level of at least any two of the following genes:
GRASP, PAK1, PPFIA1, and OTX1.

When the above reagent combination includes the above detection reagent 3), the reagent combination further includes a detection reagent for detecting the methylation level of at least any three of the following genes:
GRASP, PAK1, PPFIA1, and OTX1.

When the above reagent combination includes the above detection reagent 3), the reagent combination further includes a detection reagent for detecting the methylation level of the following four genes:
GRASP, PAK1, PPFIA1, and OTX1.

In some particular embodiments, the detection reagent for methylation level may be a detection reagent for detecting the average methylation level of the entire gene.

In some particular embodiments, the detection reagent for methylation level may also be a detection reagent for detecting the average methylation level of a gene fragment.

In some particular embodiments, the detection reagent for methylation level may also be a detection reagent for detecting the average methylation level of a gene promoter region or a fragment thereof.

In some particular embodiments, the detection reagent for methylation level may also be a detection reagent for detecting one or more methylation sites of a gene.

Those skilled in the art may select detection reagents to detect the methylation levels of GRASP, PAK1, PPFIA1, and OTX1. For example, Chinese patent CN110904225 mentions that GRASP is associated with liver cancer, Chinese patent CN106947830 mentions that PPFIA1 is associated with liver cancer, and Chinese patent CN1659287 mentions that PAK1 gene is associated with liver cancer.

In a particular embodiment, the reagent combination further includes a detection reagent for detecting the average methylation level of the entire gene of the GRASP gene.

In a particular embodiment, the reagent combination further includes a detection reagent for detecting the average methylation level of the amplified fragment of the GRASP gene in Chinese patent CN110904225:

CGCAGCCGCCACCCCTGGGCCCCCAGCGGACGAGCTGTACGCGGCGCTGGAGGAC
TATCACCCTGCCGAGCTGTACCGCGC (SEQ ID NO: 4).

In a particular embodiment, the reagent combination further includes a detection reagent for detecting the average methylation level of the following gene fragment of the GRASP gene:

CGGTCCCGACCCCGGGACCCCCTGCCGCAGCCGCCACCCCTGGGCCCCCAGCGGA
CGAGCTGTACGCGGCGCTGGAGGACTATCACCCTGCCGAGCTGTACCGCGCGCTCGCCG

TGTCCGGGGGCACCCTGCCCCGCCGAAAGGTGCGTCCCCCGCCCGCCTTCAGGATCTGC
TCAGCCCCTCTCCGACTCCCTACAGGGCCTGCTGACTCCGCAGTGCCCTCTCCTCGGCG
TCCGCGGAGTCCCCCACCTTCTTCCCCGGCCCGCTGGGTGCCTCGACTCCCCGCGTTCC
CCGCTGCTGCGAAGGCCGTGGCCCTCGCCTGCACACCGCGCCCAGGCTCG (SEQ ID NO:
5).

In some particular embodiments, the reagent combination further includes a detection reagent for detecting the methylation level of one or more of the methylation sites cg04034767 and cg00817367 of the GRASP gene.

In a particular embodiment, the reagent combination further includes a detection reagent for detecting the average methylation level of the entire gene of the PPFIA1 gene.

In a particular embodiment, the reagent combination further includes a detection reagent for detecting the average methylation level of the following gene fragment of the PPFIA1 gene:

CGACCCAGTGTTAACAGGGAATGGTTATTCTGTACGGGCATCTGAACTGAAAAGTG
AGAAGAGCGAACTTTGCCTCCTCGGCCCCTTCTCTGTGCCTGTGGCTTATGCGTGTGCCC
CTCTCCTCTTTGTCACTGCTTCCCTTGCCCTGGATGTGGTTGGTGCACTGGGGTCACCTT
AGACCACAGGAAATGTCTGGTTAACACACGAAGAGATGGAAACGCTCGCAGCCACG
(SEQ ID NO: 6).

In some particular embodiments, the detection reagent for detecting the methylation level of the PPFIA1 gene further includes a detection reagent for detecting the methylation level of one or more of the methylation sites cg14999168, cg14088196 and cg25574765 of the PPFIA1 gene.

In some particular embodiments, the reagent combination further includes a detection reagent for detecting the average methylation level of the entire gene of the PAK1 gene.

In some particular embodiments, the detection reagent for detecting the methylation level of the PAK1 gene further includes a detection reagent for detecting the methylation level of one or more of the methylation sites cg17202086, cg26996201, cg12269002 and cg18309286 of the PAK1 gene.

In some particular embodiments, the reagent combination further includes a detection reagent for detecting the average methylation level of the entire gene of the OTX1 gene.

In some particular embodiments, the detection reagent for detecting the methylation level of the OTX1 gene further includes a detection reagent for detecting the methylation level of one or more of the methylation sites cg21472506, cg23229261 and cg10122865 of the OTX1 gene.

In some particular embodiments, the detection reagent for detecting the methylation level of the ZNF397OS gene further includes a detection reagent for detecting the methylation level of one or more of the methylation sites cg27249419, cg16657538 and cg00487232 of the ZNF397OS gene.

In a preferred embodiment, the detection reagent for detecting the methylation level of the ZNF397OS gene further includes a detection reagent for detecting the methylation level of one or more fragments covering the methylation site cg16657538 of the ZNF397OS gene.

By using the reagent combination of the above resolutions, a liver cancer may be detected with higher sensitivity and better specificity; clinically, it may be detected sensitively and specifically in the early stage of liver malignancy.

In some embodiments, the methylation level of the corresponding gene present in a sample may be detected by using the detection reagents of the present invention.

In the present invention, the "sample" is a biological sample collected from an individual. Specifically, for example, it is selected from the group consisting of cell line, histological section, tissue biopsy/paraffin-embedded tissue, body fluid, feces, colonic effluent, urine, plasma, serum, whole blood, isolated blood-cells, cells isolated from blood, or a combination thereof.

Preferably, the "sample" of the present invention is plasma, i.e., cell-free DNA in plasma.

Cell-free DNA in plasma may be used to detect tumors, and has the characteristics of less harm to patients and good specificity. However, due to its extremely low content in plasma, it generally suffers from low sensitivity when being used for cancer detection. By using the detection reagent of the present invention, the cell-free DNA in the plasma may be used as a sample for detection, which has high sensitivity and specificity.

In the present invention, "detection reagent" refers to a reagent for detecting the methylation level of a gene in a sample, wherein the methylation level is detected by amplification-sequencing, chip, and methylation fluorescence quantitative PCR.

In some particular embodiments, the detection reagents include, but are not limited to nucleic acid primer and Tag sequence for sequencing, for detecting methylation level by amplification-sequencing.

In a particular embodiment, the amplification-sequencing is performed by subjecting the nucleic acid in the sample to bisulfite treatment, and constructing a pre-library, then constructing a final library, and finally performing sequencing evaluation.

In some particular embodiments, the detection reagent include, but is not limited to a chip, wherein the chip is a methylation chip having a probe specifically binding to the methylated region. The chip may be, for example, Human CpG Island Microarrays and Human DNA Methylation Microarrays from Agilent; Infinium HumanMethylation27 BeadChip, Infinium HumanMethylation450 BeadChip and GoldenGate Methylation Assay from Illumina; and Human DNA Methylation 2.1M Deluxe Promoter Array, Human DNA Methylation Array from Roche NimbleGen; et al., for measuring methylation level by chip.

In some particular embodiments, detection reagents include, but are not limited to nucleic acid primer and nucleic acid probe for measuring methylation level by quantitative PCR for methylation.

Further, the detection reagents also include an internal standard primer and an internal standard probe.

In some particular embodiments, the above reagent combination may also include other reagents, particularly, for example, various reagents required for preliminary or pretreatment of a sample. For example, sample release agent for extracting sample nucleic acid, purifying agent for purifying sample nucleic acid, bisulfite or bisulphite used for transformation, etc.

Further, the above reagent combination also includes a reagent for extracting plasma cell-free DNA.

In a second aspect, the present invention provides use of the above reagent combination in the preparation of a kit for detecting a liver cancer.

Further, the present invention provides use of the above reagent combination in preparing a kit for detecting a liver cancer by using plasma cell-free DNA.

In a third aspect, the present invention provides a kit for detecting a liver cancer, including the reagent combination as described above.

Further, the kit also includes, but is not limited to, at least one selected from the group consisting of a reagent for extracting nucleic acid, a reagent for purifying nucleic acid, bisulfite, T4 polynucleotide kinase, and T4 ligase.

Further, the reagent for extracting nucleic acid is a reagent for extracting tissue DNA or plasma cell-free DNA.

Further, the reagent for extracting nucleic acid is a reagent for extracting plasma cell-free DNA.

### BRIEF DESCRIPTION OF DRAWINGS

Fig. 1 shows the ROC diagram of ZNF397OS single gene for identification of cancer and non-cancer in peripheral blood cell-free DNA samples;
Fig. 2 shows the methylation levels of ZNF397OS gene in different groups of peripheral blood cell-free DNA samples;
Fig. 3 shows the methylation levels of the methylation sites of ZNF397OS gene in different groups of tissue samples;
Fig. 4 shows the methylation levels of methylation sites outside the target region of ZNF397OS gene (as comparative examples) in different groups of tissue samples;
Fig. 5 is the ROC diagram of PAK1 methylation sites for identification of cancer and non-cancer in cell-free DNA samples of peripheral blood;
Fig. 6 shows the methylation levels of PAK1 gene methylation sites in different groups in cell-free DNA samples of peripheral blood;
Fig. 7 shows the methylation levels of PAK1 gene methylation sites in different groups of tissue samples;
Fig. 8 shows the methylation levels of methylation sites outside the target region of PAK1 gene (as comparative examples) in different groups of tissue samples;
Fig. 9 is the ROC diagram of OTX1 gene methylation sites for identification of cancer and non-cancer in cell-free DNA samples of peripheral blood;
Fig. 10 shows the methylation levels of OTX1 gene methylation sites in different groups of peripheral blood cell-free DNA samples;
Fig. 11 is a comparison of methylation levels of OTX1 gene methylation sites in different groups of tissue samples;
Fig. 12 is a comparison of methylation levels of methylation sites outside the target region of OTX1 gene (as comparative examples) in different groups of tissue samples.

### DETAILED DESCRIPTION OF EMBODIMENTS

The present invention will be described in detail below with reference to particular embodiments and examples, and the advantages and various effects of the present invention will be more clearly presented therefrom. It should be understood by those skilled in the art that these particular embodiments and examples are used to illustrate the present invention, but not to limit it.

### Example 1: Screening of Methylated Genes

In present invention, the methylation data of a total of 785 cancer tissues, 461 para-cancer tissues or normal control tissues, and 656 healthy whole blood samples are collected from the TCGA database (https://tcga.xenahubs.net) of the UCSC Xena website and the GEO database of the American National Center for Biotechnology Information (NCBI). Differential analysis of the data of the liver cancer and control is performed, annotating the physical location and gene information of the differential sites. In order to ensure that the screening fragments have consistent methylation levels, the screening of methylated gene fragments needs to meet the following requirements: 1) the selected gene fragments are required to have no less than 2 adjacent sites with consistent methylation levels; 2) differential analysis is performed between the liver cancer and para-cancer tissue or normal control tissue, and methylation gene fragments with high consistency and difference in liver cancer samples are selected as candidate target genes; 3) differential analysis on the methylation detection data of whole blood of liver cancer and healthy samples, and the gene fragments that are differentially hypermethylated in liver cancer are selected; 4) finally, the methylation sites are analyzed one by one, thereby obtaining the candidate methylation sites.

### Example 2: Detection of Gene Methylation Levels in Clinical Samples

10ml of peripheral blood for each sample is collected for detection and analysis of the methylation level of DNA methylation markers in the samples. The experimental procedure is as follows:

### 1. Sample Preparation

The sample of the present invention is prepared by extracting 4ml of plasma with MagMAX^{™} Cell-Free DNA Isolation Kit, and eluting with 45µL of eluent. The extracted cell-free nucleic acids must meet the following quality control conditions: the total amount of extracted nucleic acids is greater than 20ng.

### 2. Library Preparation

In the present invention, all cell-free nucleic acids qualified for quality control are treated with bisulfite by using EZ DNA Methylation-Lightning^{™} Kit (Zymo Research, Irvine, CA, USA). Subsequently, the sample DNA after bisulfite treatment is used to construct a pre-library by the single-strand library construction method. After passing the quality inspection of the pre-library, the target region is captured and enriched by liquid chip hybridization to complete the construction of the final library.

Construction steps of pre-library: 1) phosphorylation: T4 polynucleotide kinase is used to phosphorylate the 5'-end of the bisulfite-treated DNA; 2) SS1 ligation: T4 DNA Ligase (Rapid) is used to ligate the SS1 linker to the 5'-end of phosphorylated DNA; 3) nucleic acid purification: 2 volumes of Agencourt AMPure XP system (Beckman CouLter, CA, USA) is used to remove the remaining linkers; 4) SS2 ligation: T4 DNA Ligase (Rapid) is used to ligate the SS2 linker to the 3'-end of phosphorylated DNA; 5) nucleic acid purification: 2 volumes of Agencourt AMPure XP system (Beckman CouLter, CA, USA) is used to remove the remaining linkers; 6) amplification: NEBNext Q5U Master Mix, primer1.0 (universal primers) and Bacard sequences are used to amplify the nucleic acids in the previous step; 7) nucleic acid purification: 1.2 volume of Agencourt AMPure XP system (Beckman CouLter, CA, USA) is used to remove primer dimers and excess primers; 8) quality inspection: the pre-library after purification is checked the total amount of the library by Qubit^{®} dsDNA HS Assay Kit (Life Technologies, CA, USA), and the quality inspection of the library fragment distribution is performed by LabChip GXII Touch.

Capture steps of chip (Twist Bioscience) hybridization: 1) chip hybridization: 1.5 µg of the library mixed well after passing the quality inspection is subjected to vacuum concentration into powder in advance, then mixing well with Panel, Hybridization Mix, Blocker Solution, Universal Blockers, and Hybridization Enhancer reagents (the reagents used for chip hybridization are all provided by Twist Bioscience), placing in a PCR amplifier to incubate for 16 h overnight at 70 degrees (the temperature of the hot lid is 85 degrees); 2) magnetic bead capture: Streptavidin Binding Buffer is used to wash the capture magnetic beads 3 times in advance, adding the hybridized product to the capture magnetic beads to incubate for 30 min, washing once with Wash Buffer I, washing 3 times with Wash Buffer 2, then eluting with 42 µl of ultra-pure water; 3) amplification: KAPA HiFi HotStart ReadyMix and universal primers are used to amplify the captured library; 4) purification: 1 volume of Agencourt AMPure XP system (Beckman CouLter, CA, USA) is used to remove primer dimers and excess primers.

As for the purified library, the quality inspection of the total amount of nucleic acid, fragment distribution and primer-dimer ratio in the library is performed by Qubit^{®} dsDNA HS Assay Kit (Life Technologies, CA, USA) and LabChip GXII Touch.

### 3. Sequencing

The libraries after passing the quality inspection of the total amount of library, fragment size distribution of the amplified products and primer-dimer ratio are used as the libraries to be tested and mixed according to the mass of 1:1, Qubit^{®} dsDNA HS Assay Kit (Life Technologies, CA, USA) is used to perform accurate quantification of the mixed library, the library is denatured and diluted, then sequencing on the NextSeq500 desktop sequencer by using PE75.

### 4. Establishment and Evaluation of Liver Cancer Classification Model

For the raw fastq data obtained by sequencing, after filtering the raw data, bismark methylation analysis software is used to perform methylation analysis on the fragments captured by the chip, thereby obtaining the methylation level of a single site of the candidate genes and the methylation level of the gene fragments. The methylation level of a single site of candidate genes and the methylation level of gene fragments are used to perform differential analysis and model construction for liver cancer and the control samples. In the present invention, liver cancer classification model is constructed and evaluated by Logistic regression analysis of the data.

### Example 3: Detection of methylation level of clinical samples by the reagent combination of the present invention

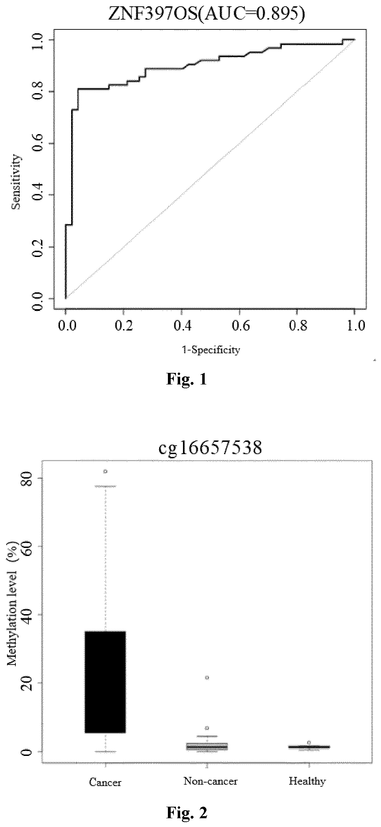

Samples from 63 patients with primary liver cancer, 25 patients with cirrhosis, 15 patients with hepatitis and 7 healthy people are collected, and the methylation level of the methylation site cg16657538 of ZNF397OS gene, and the methylation levels of the combinations of the ZNF397OS gene and the rest of the genes in the samples are detected and analyzed according to the method described in Example 2, thereby verifying the effect of detecting a liver cancer. The test results are shown in Table 1, Fig. 1 and Fig. 2.

**Table 1: Prediction performance of the combinations of ZNF397OS gene and other genes in the Logistics liver cancer classification model**

| | Data of the tissue | | | Data of the cell-free DNA | | |
|---|---|---|---|---|---|---|
| Gene combination | AUC | Sensitivity | Specificity | AUC | Sensitivity | Specificity |
| 5 | 0.9410 | 0.9100 | 0.9500 | 0.8948 | 0.7619 | 0.9574 |
| 1+5 | 0.9603 | 0.9185 | 0.9393 | 0.8997 | 0.8095 | 0.9574 |
| 2+5 | 0.9396 | 0.8994 | 0.9176 | 0.8862 | 0.8095 | 0.8936 |
| 3+5 | 0.9438 | 0.8994 | 0.9197 | 0.8845 | 0.8413 | 0.9362 |
| 4+5 | 0.9598 | 0.9134 | 0.9501 | 0.9206 | 0.873 | 0.9787 |
| 1+2+5 | 0.9679 | 0.9261 | 0.9544 | 0.9098 | 0.8413 | 0.9787 |
| 1+3+5 | 0.9647 | 0.9236 | 0.9631 | 0.9119 | 0.8254 | 0.9787 |
| 1+4+5 | 0.9663 | 0.9325 | 0.961 | 0.9348 | 0.873 | 0.9787 |
| 2+3+5 | 0.944 | 0.8981 | 0.9219 | 0.8855 | 0.8413 | 0.8936 |
| 2+4+5 | 0.9634 | 0.9134 | 0.9436 | 0.9237 | 0.8571 | 0.9149 |
| 3+4+5 | 0.9667 | 0.921 | 0.9544 | 0.9254 | 0.8571 | 0.9362 |
| 1+2+3+5 | 0.9682 | 0.9248 | 0.961 | 0.9092 | 0.8413 | 0.9787 |
| 1+2+4+5 | 0.9723 | 0.9414 | 0.9653 | 0.9433 | 0.8889 | 0.9574 |
| 1+3+4+5 | 0.9719 | 0.9338 | 0.9675 | 0.9331 | 0.873 | 0.9787 |
| 2+3+4+5 | 0.9669 | 0.9197 | 0.9544 | 0.9264 | 0.8571 | 0.9149 |
| 1+2+3+4+5 | 0.9739 | 0.9376 | 0.9696 | 0.9541 | 0.8889 | 0.9574 |

Wherein, 1 represents the GRASP gene (detecting the average methylation level of the fragment represented by SEQ ID NO: 5); 2 represents the PAK1 gene (detecting the average methylation of the fragment containing four methylation sites cg17202086, cg26996201, cg12269002 and cg18309286); 3 represents the PPFIA1 gene (detecting the average methylation level of the fragment represented by SEQ ID NO: 6); 4 represents the OTX1 gene (detecting the average methylation of the fragment containing three methylation sites cg21472506, cg23229261 and cg10122865); and 5 represents the ZNF397OS gene (detecting the average methylation level of the fragment containing methylation site cg16657538).

As can be seen from Table 1 that, in tissue samples, all reagent combinations are able to predict liver cancer with a specificity of at least 0.9176 and a sensitivity of 0.8981 and an area under the curve of 0.9396. while in plasma cell-free DNA samples, all reagent combinations are able to predict liver cancer with a specificity of at least 0.8936 and a sensitivity of 0.7619 and an area under the curve of 0.8845. Therefore, the reagent combinations of the present invention have a good predictive effect on liver cancer, and in particular when plasma cell-free DNA is used as a sample, they have excellent specificity and sensitivity.

### Comparative Example 1

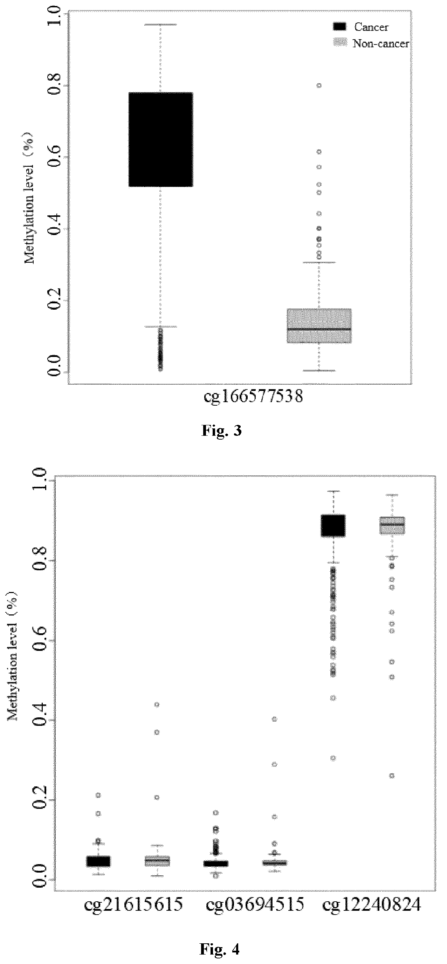

In order to investigate whether other methylation sites on ZNF397OS gene may uniformly be used as markers for detecting a liver cancer, another methylation site in ZNF397OS gene is selected as a comparative example (all of which are located outside the currently selected target fragment of ZNF397OS gene), comparing the methylation differences in cancer and non-cancer samples; the results are shown in Fig. 3 and Fig. 4. It can be seen from Fig. 3 and Fig. 4 that, the methylation sites upstream and downstream of the selected target fragment of ZNF397OS gene have no significant difference between liver cancer and non-cancer tissues, and are not suitable to be used as markers for detecting a liver cancer.

### Comparative Example 2

In order to investigate whether the ZNF397OS gene may be used as a marker for detecting a liver cancer, the genes PLAC8 and ATXN1, which are closely related to liver cancer known in the art, are further selected (see Xu RH, Wei W, Krawczyk M, Wang W, Luo H, Flagg K, et al. al. Circulating tumor DNA methylation markers for diagnosis and prognosis for diagnosis and prognosis of hepatocellular carcinoma. Nature Materials, 2017, and Chinese patent CN106947830B). The detection is also performed according to the method of the above example, and the detection results are shown in Table 3 below.

**Table 3: Prediction performance of the comparative genes and their combinations in the Logistics liver cancer classification model**

| | Data of the tissue | | | Data of the cell-free DNA | | |
|---|---|---|---|---|---|---|
| Gene combination | AUC | Sensitivity | Specificity | AUC | Sensitivity | Specificity |
| 6 | 0.578 | 0.944 | 0.004 | 0.750 | 0.683 | 0.681 |
| 7 | 0.858 | 0.813 | 0.711 | 0.722 | 0.746 | 0.574 |
| 6+7 | 0.872 | 0.827 | 0.722 | 0.883 | 0.810 | 0.809 |

Wherein, 6 represents the PLAC8 gene (detecting the methylation level of cg11606215 of this gene), and 7 represents the ATXN1 gene (detecting the methylation level of cg24067911 of this gene).

As can be seen from Table 3 that, in tissue samples, the highest area under the curve of a single comparative gene is 0.858, while in cell-free DNA samples, the highest area under the curve is 0.75, which are lower than the area under the curve of ZNF397OS of the present invention. The area under the curve of the combination is also lower than the value of the area under the curve of the combination of the agents of the present invention.

### Example 4: Detection of Methylation Level of Clinical Samples by the Reagent Combination of the Present Invention

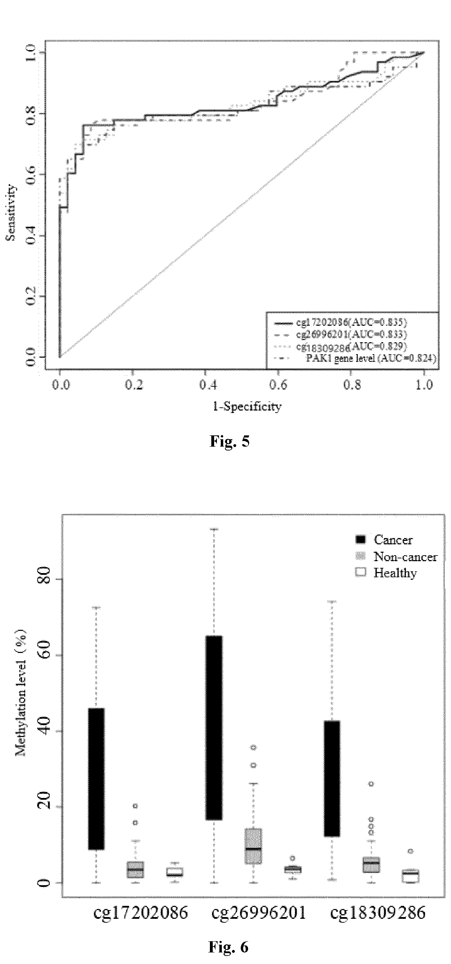

Samples from 63 patients with primary liver cancer, 25 patients with liver cirrhosis, 15 patients with hepatitis, and 7 healthy people are collected, and the methylation sites of PAK1 gene in the samples are detected and analyzed according to the method described in Example 2, thereby verifying their effect in detecting a liver cancer. The diagnosis of hepatocellular carcinoma, liver cirrhosis, hepatitis and healthy people is based on the final hospital pathological diagnosis. The test results are shown in Table 4, Fig. 5 and Fig. 6.

**Table 4: Prediction performance of methylation sites of PAK1 gene in the Logistics liver cancer classification model**

| | Data of the tissue | | | Data of the cell-free DNA | | |
|---|---|---|---|---|---|---|
| Methylation site | AUC | Sensitivity | Specificity | AUC | Sensitivity | Specificity |
| cg17202086 | 0.8607 | 0.865 | 0.655 | 0.8352 | 0.7619 | 0.8936 |
| cg26996201 | 0.8194 | 0.903 | 0.360 | 0.8333 | 0.7778 | 0.8936 |
| cg18309286 | 0.8729 | 0.871 | 0.625 | 0.8286 | 0.7460 | 0.8511 |
| cg17202086+ cg26996201 | 0.853 | 0.843 | 0.662 | 0.833 | 0.778 | 0.894 |
| cg17202086+ cg18309286 | 0.876 | 0.870 | 0.651 | 0.838 | 0.778 | 0.872 |
| cg26996201+ cg18309286 | 0.884 | 0.870 | 0.716 | 0.837 | 0.794 | 0.894 |
| cg17202086+ cg26996201+cg18309286 | 0.887 | 0.868 | 0.729 | 0.839 | 0.778 | 0.872 |

As can be seen from Table 4 that, in tissues, each methylation site of the PAK1 gene may predict liver cancer with a specificity of at least 0.360, a sensitivity of 0.843, and an area under the curve of 0.8194. while in plasma cell-free DNA, all reagent combinations are able to predict a liver cancer with a specificity of at least 0.8511, a sensitivity of 0.7460, and an area under the curve of 0.833.

### Example 5: Detection of methylation Level of Clinical Samples by the Reagent Combinations of the Present Invention

Samples from 63 patients with primary liver cancer, 25 patients with liver cirrhosis, 15 patients with hepatitis and 7 healthy people are collected, and the methyl levels of the combinations of PAK1 gene and other genes in the samples are detected and analyzed according to the method described in Example 2, thereby verifying their effect on detecting a liver cancer. The test results are shown in Table 5 below.

**Table 5: Prediction performance of the combinations of PAK1 gene and other genes in the Logistics liver cancer classification model**

| | Data of the tissue | | | Data of the cell-free DNA | | |
|---|---|---|---|---|---|---|
| Gene combination | AUC | Sensitivity | Specificity | AUC | Sensitivity | Specificity |
| 1+2 | 0.9508 | 0.9096 | 0.961 | 0.8625 | 0.7937 | 0.9787 |
| 2+5 | 0.9396 | 0.8994 | 0.9176 | 0.8862 | 0.8095 | 0.8936 |
| 2+3 | 0.8944 | 0.893 | 0.6594 | 0.8578 | 0.8095 | 0.9149 |
| 2+4 | 0.9398 | 0.8904 | 0.9024 | 0.9132 | 0.8571 | 0.9149 |
| 1+2+5 | 0.9679 | 0.9261 | 0.9544 | 0.9098 | 0.8413 | 0.9787 |
| 1+2+3 | 0.9523 | 0.9108 | 0.9675 | 0.874 | 0.7937 | 0.9787 |
| 1+2+4 | 0.963 | 0.9223 | 0.9631 | 0.9301 | 0.873 | 0.9787 |
| 2+3+5 | 0.944 | 0.8981 | 0.9219 | 0.8855 | 0.8413 | 0.8936 |
| 2+4+5 | 0.9634 | 0.9134 | 0.9436 | 0.9237 | 0.8571 | 0.9149 |
| 2+3+4 | 0.9461 | 0.8955 | 0.9067 | 0.9176 | 0.8571 | 0.9149 |
| 1+2+3+5 | 0.9682 | 0.9248 | 0.961 | 0.9092 | 0.8413 | 0.9787 |
| 1+2+4+5 | 0.9723 | 0.9414 | 0.9653 | 0.9433 | 0.8889 | 0.9574 |
| 1+2+3+4 | 0.9657 | 0.9248 | 0.9696 | 0.9362 | 0.873 | 0.9787 |
| 2+3+4+5 | 0.9669 | 0.9197 | 0.9544 | 0.9264 | 0.8571 | 0.9149 |
| 1+2+3+4+5 | 0.9739 | 0.9376 | 0.9696 | 0.9541 | 0.8889 | 0.9574 |

Wherein, 1 represents the GRASP gene (detecting the average methylation level of the fragment represented by SEQ ID NO: 5); 2 represents the PAK1 gene (detecting the methylation levels of the three methylation sites cg17202086, cg26996201, and cg18309286); 3 represents the PPFIA1 gene (detecting the average methylation level of the fragment represented by SEQ ID NO: 6); 4 represents the OTX1 gene (detecting the methylation levels of three methylation sites cg21472506, cg23229261 and cg10122865); and 5 represents the ZNF397OS gene (detecting the methylation level of the methylation site cg16657538).

It can be seen in Table 5 that, in tissue samples, all reagent combinations are able to predict a liver cancer with a specificity of at least 0.9176, a sensitivity of 0.8904, and an area under the curve of 0.8944; while in plasma cell-free DNA samples, all reagent combinations are able to predict a liver cancer with a specificity of at least 0.7937, a sensitivity of 0.8578, and an area under the curve of 0.8578. Therefore, the reagent combinations of the present invention have a good predictive effect on liver cancer, and in particular when plasma cell-free DNA is used as a sample, they have excellent specificity and sensitivity.

### Comparative Example 3

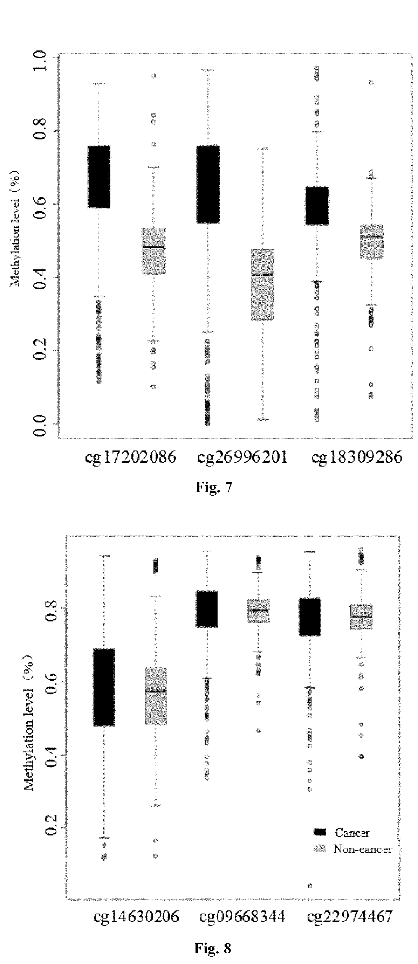

In order to investigate whether other methylation sites on PAK1 gene may uniformly be used as markers for detecting a liver cancer, the methylation sites upstream and downstream of the selected target fragment in PAK1 gene are selected, comparing their methylation differences in cancer and non-cancer samples; the results are shown in Fig. 7 and Fig. 8. It can be seen from Fig. 7 and Fig. 8 that, the methylation sites upstream and downstream of the selected target fragment in the PAK1 gene have no significant difference between cancer and non-cancer tissues, and are not suitable to be used as markers for detecting a liver cancer.

### Comparative Example 4

In order to investigate whether the PAK1 gene may be used as a marker for detecting a liver cancer, we further selected the common liver cancer-related genes PLAC8 and ATXN1 known in the art (see Xu RH, Wei W, Krawczyk M, Wang W, Luo H, Flagg K, et al. al. Circulating tumor DNA methylation markers for diagnosis and prognosis for diagnosis and prognosis of hepatocellular carcinoma. Nature Materials, 2017, and Chinese patent CN106947830B). The detection is also performed according to the method of the above example, and the detection results are shown in Table 6 below.

**Table 6: Prediction performance of the comparative genes and their combinations in the Logistics liver cancer classification model**

| | Data of the tissue | | | Data of the cell-free DNA | | |
|---|---|---|---|---|---|---|
| Gene combination | AUC | Sensitivity | Specificity | AUC | Sensitivity | Specificity |
| 6 | 0.578 | 0.944 | 0.004 | 0.750 | 0.683 | 0.681 |
| 7 | 0.858 | 0.813 | 0.711 | 0.722 | 0.746 | 0.574 |
| 6+7 | 0.872 | 0.827 | 0.722 | 0.883 | 0.810 | 0.809 |

Wherein, 6 represents the PLAC8 gene (detecting the methylation level of cg11606215 of this gene), and 7 represents the ATXN1 gene (detecting the methylation level of cg24067911 of this gene).

It can be seen from Table 6 that, in tissues, the highest area under the curve of the single control gene is 0.858, and the highest area under the curve of the cell-free DNA is 0.75, which are lower than the area under the curve of the PAK1 gene of the present invention. The area under the curve of the combination is also lower than the value of the area under the curve of the combination of the agents of the present invention.

### Example 6: Detection of methylation Level of Clinical Samples by the Reagent Combination of the Present invention

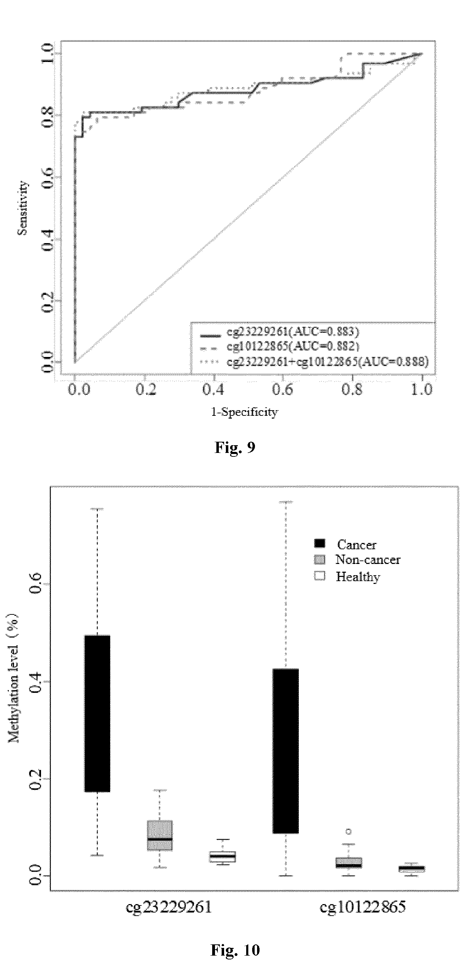

Samples from 63 patients with primary liver cancer, 25 patients with liver cirrhosis, 15 patients with hepatitis, and 7 healthy people are collected, and the methylation level of the OTX1 gene in the samples is detected and analyzed according to the method described in Example 2, thereby verifying its effect on detecting a liver cancer. Diagnosis of hepatocellular carcinoma, cirrhosis, hepatitis and healthy people is based on the final hospital pathological diagnosis. The test results are shown in Table 7, Fig. 9 and Fig. 10.

**Table 7: Prediction performance of OTX1 gene methylation sites in the Logistics liver cancer classification model**

| | Data of the tissue | | | Data of the cell-free DNA | | |
|---|---|---|---|---|---|---|
| Methylation site | AUC | Sensitivity | Specificity | AUC | Sensitivity | Specificity |
| cg23229261 | 0.929 | 0.884 | 0.885 | 0.8828 | 0.7937 | 0.9574 |
| cg10122865 | 0.914 | 0.860 | 0.846 | 0.8823 | 0.8095 | 0.8298 |
| cg23229261+ cg10122865 | 0.934 | 0.882 | 0.885 | 0.888 | 0.810 | 0.957 |
| cg21472506+ cg23229261 | 0.941 | 0.890 | 0.892 | 0.887 | 0.778 | 0.957 |
| cg21472506+ cg10122865 | 0.938 | 0.887 | 0.907 | 0.889 | 0.810 | 0.936 |
| cg21472506+ cg23229261+ cg10122865 | 0.942 | 0.887 | 0.896 | 0.888 | 0.794 | 0.957 |

It can be seen from Table 7 that, in tissues, each methylation site of the OTX1 gene may predict a liver cancer with a specificity of at least 0.846, a sensitivity of 0.860, and an area under the curve of 0.914; while in plasma cell-free DNA, all reagent combinations are able to predict a liver cancer with a specificity of at least 0.8298, a sensitivity of 0.778, and an area under the curve of 0.8823.

### Example 7: Detection of methylation Level of Clinical Samples by the Reagent Combination of the Present Invention

Samples from 63 patients with primary liver cancer, 25 patients with liver cirrhosis, 15 patients with hepatitis and 7 healthy people are collected, and the methylation levels of the combinations of OTX1 gene and other genes in the samples are detected and analyzed according to the method described in Example 2, thereby verifying their effect on detecting a liver cancer. The test results are shown in Table 8 below:

**Table 8: Prediction performance of the combinations of OTX1 gene and other genes in the Logistics liver cancer classification model**

| | Data of the tissue | | | Data of the cell-free DNA | | |
|---|---|---|---|---|---|---|
| Gene combination | AUC | Sensitivity | Specificity | AUC | Sensitivity | Specificity |
| 1+4 | 0.9625 | 0.9236 | 0.9631 | 0.9237 | 0.873 | 0.9787 |
| 2+4 | 0.9398 | 0.8904 | 0.9024 | 0.9132 | 0.8571 | 0.9149 |
| 3+4 | 0.9433 | 0.8968 | 0.9024 | 0.9169 | 0.873 | 0.8723 |
| 4+5 | 0.9598 | 0.9134 | 0.9501 | 0.9206 | 0.873 | 0.9787 |
| 1+2+4 | 0.963 | 0.9223 | 0.9631 | 0.9301 | 0.873 | 0.9787 |
| 1+3+4 | 0.966 | 0.9248 | 0.9696 | 0.922 | 0.873 | 1 |
| 1+4+5 | 0.9663 | 0.9325 | 0.961 | 0.9348 | 0.873 | 0.9787 |
| 2+3+4 | 0.9461 | 0.8955 | 0.9067 | 0.9176 | 0.8571 | 0.9149 |
| 2+4+5 | 0.9634 | 0.9134 | 0.9436 | 0.9237 | 0.8571 | 0.9149 |
| 3+4+5 | 0.9667 | 0.921 | 0.9544 | 0.9254 | 0.8571 | 0.9362 |
| 1+2+3+4 | 0.9657 | 0.9248 | 0.9696 | 0.9362 | 0.873 | 0.9787 |
| 1+2+4+5 | 0.9723 | 0.9414 | 0.9653 | 0.9433 | 0.8889 | 0.9574 |
| 1+3+4+5 | 0.9719 | 0.9338 | 0.9675 | 0.9331 | 0.873 | 0.9787 |
| 2+3+4+5 | 0.9669 | 0.9197 | 0.9544 | 0.9264 | 0.8571 | 0.9149 |
| 1+2+3+4+5 | 0.9739 | 0.9376 | 0.9696 | 0.9541 | 0.8889 | 0.9574 |

Wherein, 1 represents the GRASP gene (detecting the average methylation level of the fragment represented by SEQ ID NO: 5); and 2 represents the PAK1 gene (detecting the methylation levels of four methylation sites cg17202086, cg26996201, cg12269002, and cg18309286); 3 represents the PPFIA1 gene (detecting the average methylation level of the fragment represented by SEQ ID NO: 6); 4 represents the OTX1 gene (detecting the average methylation levels of the three methylation sites cg21472506, cg23229261, cg10122865); 5 represents the ZNF397OS gene (detecting the methylation level of the methylation site cg16657538).

It can be seen from the table that, in tissue samples, all reagent combinations are able to predict a liver cancer with a specificity of at least 0.9024, a sensitivity of 0.8904 and an area under the curve of 0.9398; while in plasma cell-free DNA samples, all reagent combinations are able to predict a liver cancer with a specificity of at least 0.8723, a sensitivity of 0.8571, and an area under the curve of 0.9132. Therefore, the reagent combinations of the present invention have a good predictive effect on detecting a liver cancer, and in particular when plasma cell-free DNA is used as a sample, they have excellent specificity and sensitivity.

### Comparative Example 5

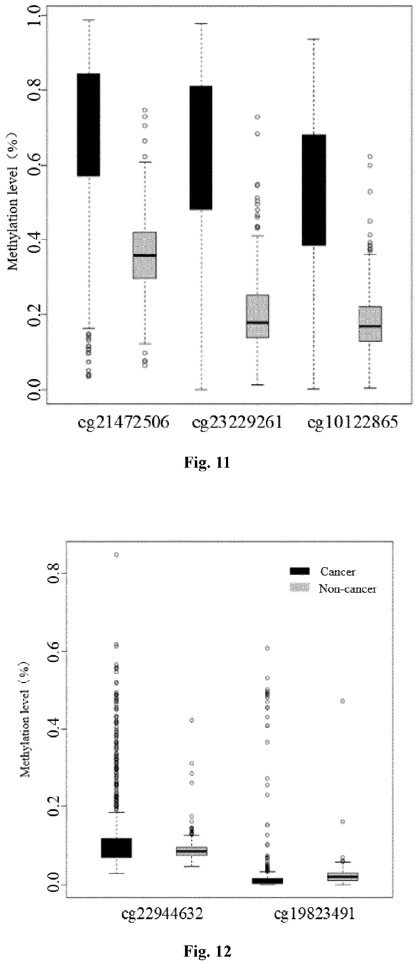

In order to investigate whether other methylation sites on OTX1 gene may uniformly be used as markers for the detection of liver cancer, the methylation sites upstream of the selected target fragment in OTX1 gene are selected, comparing the methylation differences in cancer and non-cancer samples; and the results are shown in Fig. 11 and Fig. 12. It can be seen from Fig. 11 and Fig. 12 that, the methylation sites upstream of the selected target fragment in the OTX1 gene have no significant difference between cancerous and non-cancerous tissues, and they are not suitable to be used as markers for detecting a liver cancer.

### Comparative Example 6

In order to investigate whether the OTX1 gene may be used as a marker for detecting a liver cancer, we further select the common liver cancer-related genes PLAC8 and ATXN1 known in the art (see Xu RH, Wei W, Krawczyk M, Wang W, Luo H, Flagg K, et al. al. Circulating tumor DNA methylation markers for diagnosis and prognosis for diagnosis and prognosis of hepatocellular carcinoma. Nature Materials, 2017, and Chinese patent CN106947830B). The detection is also performed according to the method of the above example, and the detection results are shown in Table 9 below.

**Table 9: Prediction performance of the comparative genes and their combinations in the Logistics liver cancer classification model**

| | Data of the tissue | | | Data of the cell-free DNA | | |
|---|---|---|---|---|---|---|
| Gene combination | AUC | Sensitivity | Specificity | AUC | Sensitivity | Specificity |
| 6 | 0.578 | 0.944 | 0.004 | 0.750 | 0.683 | 0.681 |
| 7 | 0.858 | 0.813 | 0.711 | 0.722 | 0.746 | 0.574 |
| 6+7 | 0.872 | 0.827 | 0.722 | 0.883 | 0.810 | 0.809 |

Wherein, 6 represents the PLAC8 gene (detecting the methylation level of cg11606215 of this gene), and 7 represents the ATXN1 gene (detecting the methylation level of cg24067911 of this gene).

It can be seen from Table 9 that, in the tissue samples, the highest area under the curve of the single control gene is 0.858, while in the cell-free DNA samples, the highest area under the curve is 0.75, which are lower than the area under the curve of the OTX1 gene of the present invention. The area under the curve of the gene combination model is also lower than the value of the area under the curve of the reagent-related combination model of the present invention.

## Claims

1. A reagent combination for detecting a liver cancer, comprising any one of the following detection reagents:
1) a detection reagent for detecting the methylation level of at least one of the following methylation sites of the PAK1 gene: cg17202086, cg26996201, and cg18309286;
2) a detection reagent for detecting the methylation level of at least one of the following methylation sites of the OTX1 gene: cg23229261 and cg10122865; and
3) a detection reagent for detecting the methylation level of cg16657538 methylation site of ZNF397OS gene.

2. The reagent combination according to claim 1, wherein when the reagent combination comprises the above detection reagent 1), the reagent combination further comprises a detection reagent for detecting the methylation level of at least one of the following genes: GRASP, ZNF397OS, PPFIA1 , and OTX1.

3. The reagent combination according to claim 1, wherein when the reagent combination comprises the above detection reagent 1), the reagent combination further comprises a detection reagent for detecting the methylation level of at least two of the following genes: GRASP, ZNF397OS, PPFIA1, and OTX1.

4. The reagent combination according to claims 1, wherein when the reagent combination comprises the above detection reagent 1), the reagent combination further comprises a detection reagent for detecting the methylation level of at least three of the following genes: GRASP, ZNF397OS, PPFIA1, and OTX1.

5. The reagent combination according to claim 1, wherein when the reagent combination comprises the above detection reagent 1), the reagent combination further comprises a detection reagent for detecting the methylation level of the following four genes: GRASP, ZNF397OS, PPFIA1, and OTX1.

6. The reagent combination according to claim 1, wherein when the reagent combination comprises the above detection reagent 2), the reagent combination comprises a detection reagent for detecting the methylation level of the following methylation sites of the OTX1 gene: cg23229261, cg10122865.

7. The reagent combination according to claim 1, wherein when the reagent combination comprises the above detection reagent 2), the reagent combination further comprises a detection reagent for detecting the methylation level of the methylation site cg21472506 of the OTX1 gene.

8. The reagent combination according to claim 1, wherein when the reagent combination comprises the above detection reagent 2), the reagent combination further comprises a detection reagent for detecting the methylation level of at least one of the following genes: GRASP, PAK1, PPFIA1, and ZNF397OS.

9. The reagent combination according to claim 1, wherein when the reagent combination comprises the above detection reagent 2), the reagent combination further comprises a detection reagent for detecting the methylation level of at least two of the following genes: GRASP, PAK1, PPFIA1, and ZNF397OS.

10. The reagent combination according to claim 1, wherein when the reagent combination comprises the above detection reagent 2), the reagent combination further comprises a detection reagent for detecting the methylation level of at least three of the following genes: GRASP, PAK1, PPFIA1, and ZNF397OS.

11. The reagent combination according to claim 1, wherein when the reagent combination comprises the above detection reagent 2), the reagent combination further comprises a detection reagent for detecting the methylation level of the following four genes: GRASP, PAK1, PPFIA1, and ZNF397OS.

12. The reagent combination according to claim 1, wherein when the reagent combination comprises the above detection reagent 3), the reagent combination further comprises a detection reagent for detecting the methylation level of at least one of the following genes: GRASP, PAK1, PPFIA1, and OTX1.

13. The reagent combination according to claim 1, wherein when the reagent combination comprises the above detection reagent 3), the reagent combination further comprises a detection reagent for detecting the methylation level of at least two of the following genes: GRASP, PAK1, PPFIA1, and OTX1.

14. The reagent combination according to claim 1, wherein when the reagent combination comprises the above detection reagent 3), the reagent combination further comprises a detection reagent for detecting the methylation level of at least three of the following genes: GRASP, PAK1, PPFIA1, and OTX1.

15. The reagent combination according to claim 1, wherein when the reagent combination comprises the above detection reagent 3), the reagent combination further comprises a detection reagent for detecting the methylation level of the following four genes: GRASP, PAK1, PPFIA1, and OTX1.

16. The reagent combination according to any one of claims 1-15, wherein the detection reagent is any one or more selected from the group consisting of: nucleic acid primer, sequencing Tag sequence, methylation chip, and nucleic acid probe.

17. The reagent combination according to any one of claims 1-15, wherein the reagent combination further comprises a reagent for extracting plasma cell-free DNA.

18. Use of the reagent combination according to any one of claims 1-17 in the preparation of a kit for detecting a liver cancer.

19. Use of the reagent combination according to any one of claims 1-17 in the preparation of a kit for detecting a liver cancer by using plasma cell-free DNA.

20. A kit for detecting a liver cancer, comprising the reagent combination according to any one of claims 1-17.

21. The kit according to claim 20, wherein the kit further comprises other reagents, such as a reagent for extracting nucleic acid, preferably at least one selected from the group consisting of a reagent for extracting plasma cell-free DNA, a reagent for purifying nucleic acid, bisulfite, T4 polymer nucleotide kinase and T4 ligase.
